# EUROPÄISCHE PATENTANMELDUNG

(11) **EP 1 746 155 A1**
(43) Veröffentlichungstag der Anmeldung: **24.01.2007**
(21) Anmeldenummer: 06120633.0
(22) Anmeldetag: 14.09.2002
(51) Int. Cl.: C12N 9/96, A23K 1/165

(54) **Verfahren zur Herstellung oeliger Suspensionen wasserloeslicher Enzyme**

(30) Priorität: 25.09.2001 DE 10147035
(62) Teilanmeldung aus: 02777104.7
(71) Anmelder: BASF AKTIENGESELLSCHAFT, 67056 Ludwigshafen (DE)
(72) Erfinder: Habich, Andreas, 67346 Speyer (DE); Runge, Frank, 67159 Friedelsheim (DE); Braun, Jörg, 76879 Essingen (DE); Soerensen, Preben, 2610 Roedoure (DK); Heinzl, Wolfgang, 67157 Wachenheim (DE)

(57) **Zusammenfassung**

Die Erfindung betrifft ölige Suspensionen mindestens eines wasserlöslichen Enzyms sowie ein Verfahren zur Herstellung dieser Suspensionen und deren Verwendung als Zusatz zu Tierfuttermitteln.

## Beschreibung

Die Erfindung betrifft ölige Suspensionen mindestens eines wasserlöslichen Enzyms sowie ein Verfahren zur Herstellung dieser Suspensionen und deren Verwendung als Zusatz zu Tierfuttermitteln.

Das Zumischen von wasserlöslichen Enzymen zu Futtermitteln erfolgt im Stand der Technik als Feststoff als Granulat oder Extrudat vor dem Pelletieren der Futtermittelmischung.

Dies hat den Nachteil, daß zum Erreichen ausreichender Pelletierstabilität bei höheren Pelletiertemperaturen das Aufbringen von Umhüllungen auf das Granulat oder Extrudat in einem zusätzlichen Verfahrensschritt erforderlich ist.

Weiterhin können flüßige, wäßrige Formulierungen von wasserlöslichen Enzymen nach dem Pelletieren durch Aufsprühen auf die Futtermittelpellets appliziert werden.

Diese wäßrigen Formulierungen sind kommerziell erhältlich oder können aus Instantpulvern durch Lösen in Wasser direkt hergestellt werden.

Wäßrige Formulierungen weisen in der Regel den Nachteil mikrobiologischer Stabilität und eine geringe Lagerstabilität auf.

JP 09 322 770 beschreibt stabile Enzym in Öl-Dispersionen und deren Verwendung zum Weichen von Fleisch.

Es war daher die Aufgabe der vorliegenden Erfindung, stabile flüssige Formulierungen wasserlöslicher Enzyme bereitzustellen, die die oben genannten Nachteile des Standes der Technik nicht aufweisen.

Diese Aufgabe wurde erfindungsgemäß gelöst durch ein Verfahren zur Herstellung öliger Suspensionen wasserlöslicher Enzyme, dadurch gekennzeichnet, daß man in Abwesenheit wasserlöslicher Vitamine
a) mindestens ein wasserlösliches Enzym in einem Öl, bevorzugt in mindestens einem eßbaren Öl bis auf eine mittlere Partikelgröße von 0,1 bis 100 µm mahlt oder
b) mindestens ein wasserlösliches Enzym ohne Verwendung einer kontinuierlichen Phase bis auf eine mittlere Partikelgröße von 0,1 bis 100 µm mahlt und die gemahlenen Partikel anschließend in einem Öl, bevorzugt in mindestens einem eβ-baren Öl suspendiert.

Bei den wasserlöslichen Vitaminen handelt es sich insbesondere um Ascorbinsäure und deren Salze wie Natriumascorbat sowie Vitamin C-Derivate wie Natrium-, Calcium- oder Magnesium-ascorbyl-2-monophosphat oder Calcium-ascorbyl-2-polyphosphat, Calcium-pantothenat, Panthenol, Vitamin B₁ (Thiamin) - als Hydrochlorid, Nitrat oder Pyrophosphat, Vitamin B₂ (Riboflavin) und deren Phosphate, Vitamin B₆ und Salze, Vitamin B₁₂, Biotin, Folsäure und Folsäurederivate wie Tetrahydrofolsäure, 5-Methyltetrahydrofolsäure, 5-Formyltetrahydrofolsäure, Nicotinsäure und Nicotinsäureamid.

Als wasserlösliches Vitamin sei in diesem Zusammenhang auch Vitamin K₃ (Menadion) als Natriumbisulfit genannt.

Bei den wasserlöslichen Enzymen handelt es sich insbesondere Oxidoreduktasen, Transferasen, Lyasen, Isomerasen, Ligasen und Hydrolasen. Bevorzugte Enzyme sind Hydrolasen. Beispiele für Hydrolase, daß heißt Enzyme welche eine hydrolytische Spaltung von chemischen Verbindungen bewirken sind, Esterasen, Glycosidasen, Lipasen, Phospholipasen, Etherhydrolasen, Proteasen, Amidasen, Aminidasen, Nitrilasen und Phosphatasen.

Glycosidasen umfassen sowohl endo- als auch exo-Glucosidasen, die sowohl α- als auch β-glycosidische Bindungen spalten. Typische Beispiele hierfür sind: Amylasen, Maltasen, Cellulasen, Endo-Xylanasen, β-Glucanasen, Mannanasen, Lysozyme, Galactosidasen, β-Glucuronidasen, Glucoseoxidasen, Saccharasen, Lactasen und dergeleichen.

Bevorzugt sind insbesondere nichtstärkepolysaccharidspaltende Enzyme, wie zum Beispiel Amylase, Glucanase und Xylanase sowie Phosphatasen, wie insbesondere Phytase.

Im erfindungsgemäßen Verfahren können die wasserlöslichen Enzyme auch als Kombination der beschriebenen Enzyme eingesetzt werden.

Die nachstehend beschriebenen Ausführungsformen beziehen sich daher immer auch auf die Kombination von wasserlöslichen Enzymen.

Die oben genannten wasserlöslichen Enzyme können vor der Mahlung in beliebiger Weise in fester Form vorliegen. Herstellbar sind diese Feststoffe in an sich bekannter Weise beispielsweise durch Trocknungs- oder Fällungsverfahren aus Kulturbrühen. Vorzugsweise werden diese Kulturbrühen vor der Trocknung oder Fällung filtriert oder zentrifugiert. Trocknungsverfahren sind beispielsweise Sprühtrocknungs-, Wirbelschichttrocknungs- oder Kontakttrocknungsverfahren, insbesondere Gefriertrocknungsverfahren.

Die Fällungsverfahren sind an sich bekannt, wie beispielsweise Fällung durch Na₂SO₄-Zusatz. Fällungsmethoden sind beispielsweise in "Enzyme der Tierernährung, AWT, 1997, Roonstraße 53175 Bonn" beschrieben.

Dementsprechend können die wasserlöslichen Enzyme beispielsweise als Pulver, Granulate oder Lyophyllisate vorliegen.

Bevorzugt werden die wasserlöslichen Enzyme vor der Mahlung als Feststoffe verwendet, die einen Wassergehalt kleiner 10 Gew.-% aufweisen.

Als eßbare Öle kommen in der Regel alle physiologisch unbedenklichen Öle - sowohl pflanzlichen als auch tierischen Ursprungs - in Frage, insbesondere solche Öle, die bei 20°C flüssig sind bzw. die in der Suspension bei 20°C allein oder zusammen mit anderen Ölen die flüssige Phase bilden. Bevorzugt zu nennen sind in diesem Zusammenhang Sonnenblumenöl, Palmöl, Sesamöl, Maiskeimöl, Baumwollsaatöl, Sojabohnenöl oder Erdnußöl, Ester mittelkettiger Triglyceride sowie außerdem Fischöle wie beispielsweise Makrelen-, Sprotten- oder Lachsöl. Für die Tierernährung besonders bevorzugt sind Fischöle, Maiskeimöl, Sonnenblumenöl und Erdnußöl. Für den Food-/Pharmabereich zusätzlich von Vorteil sind die Ester mittelkettiger Triglyceride.

Als eßbares Öl im Sinne der Erfindung sind auch Vitamin E, Vitamin E-Derivate oder Mischungen davon zu verstehen. Die Bezeichnung Vitamin E steht in diesem Zusammenhang für natürliches oder synthetisches α-, β-, γ- oder δ-Tocopherol, bevorzugt für natürliches oder synthetisches α-Tocopherol sowie für Tocotrienol. Vitamin E-Derivate sind z.B. Tocopheryl-C₁-C₂₀-Alkansäureester wie Tocopherylacetat oder Tocopherylpalmitat.

Vitamin E und/oder deren Derivate können dabei allein oder zusammen mit den anderen eßbaren Ölen als Dispergiermedium verwendet werden.

Die Mahlung kann in an sich bekannter Weise z.B. mit einer Kugelmühle erfolgen. Dabei wird je nach verwendetem Mühlentyp so lange gemahlen, bis die Teilchen eine mittlere Partikelgröße D[4,3] von 0,1 bis 100 µm, bevorzugt 0,2 bis 80 µm, besonders bevorzugt 0,5 bis 50 µm, ganz besonders bevorzugt 0,8 bis 40 µm., beispielsweise über Fraunhofer Beugung gemessen, aufweisen. Der Begriff D[4,3] bezeichnet den volumengewichteten mittleren Durchmesser (siehe Handbuch zu Malvern Mastersizer S, Malvern Instruments Ltd., UK).

Es kann vorteilhaft sein, bei großen Festkörpern eine Vormahlung der Enzymfeststoffe durchzuführen.

Nähere Einzelheiten zur Mahlung und den dafür eingesetzten Apparaturen finden sich u.a. in Ullmann's Encyclopedia of Industrial Chemistry, Sixth Edition, 1999, Electronic Release, Size Reduction, Kapitel 3.6.: Wet Grinding.

Bei dem erfindungsgemäßen Mahlverfahren ist es möglich, alle in der Suspension verwendeten Komponenten als Gesamtmischung zu mahlen. Es kann aber auch jede einzelne zu mahlende Komponente in hoher Konzentration in dem zu verwendenden Öl gemahlen werden. Die Endzubereitung ergibt sich dann durch eine Abmischung der jeweiligen Einzelsuspensionen.

Die erfindungsgemäße Zubereitung kann vor der Anwendung mit Fetten oder Ölen auf die jeweilige Gebrauchskonzentration verdünnt werden.

Eine besondere Ausführungsform des erfindungsgemäßen Verfahrens ist dadurch gekennzeichnet, daß die Mahlung im Schritt a) sowie die Mahlung und/oder Suspendierung im Schritt b) in Abwesenheit eines Emulgators erfolgen.

Eine weitere vorteilhafte Ausführungsform des erfindungsgemäßen Verfahrens ist dadurch gekennzeichnet, daß die Mahlung im Schritt a) sowie die Mahlung und/oder Suspendierung im Schritt b) in Abwesenheit eines Schutzkolloids erfolgen.

Trotz Fehlen der o.g. Dispergier- bzw. Formulierhilfsmittel konnten die an sich hydrophilen Enzyme - für den Fachmann unerwartet - ohne Benetzungsprobleme und Agglomeratbildung in den o.g. hydrophoben Dispergiermedien feinstgemahlen werden.

Neben der oben beschriebenen Naßmahlung, lassen sich die erfindungsgemäßen öligen Suspensionen auch durch Trockenmahlung der wasserlöslichen Enzyme und anschließendes Suspendieren der gemahlenen Partikel in mindestens einem eßbaren Öl herstellen. Als Trockenmahlung versteht man in diesem Zusammenhang eine Mahlung ohne Verwendung einer kontinuierlichen Phase.

Nähere Einzelheiten zur Trockenmahlung finden sich u.a. in Ullmann's Encyclopedia of Industrial Chemistry, Sixth Edition, 1999, Electronic Release, Size Reduction, Kapitel 3.4.

Als besonderen Vorteil hinsichtlich der Stabilität der erfindungsgemäßen öligen Dispersionen hat sich herausgestellt, wenn man die Mahlung imVerfahrensschritt a) sowie die Mahlung und/oder Suspendierung im Schritt b) in Gegenwart von Trockenmitteln durchführt. Bevorzugt sind dabei Trockenmittel, ausgewählt aus der Gruppe, bestehend aus Alkali- und Erdalkalisulfaten wie Natrium-, Calcium- und Magnesiumsulfat, Alkali- und Erdalkalichloriden wie Natrium-, Calcium- und Magnesiumchlorid und Kieselgel. Als ganz besonders bevorzugtes Trockenmittel ist CaCl₂ zu nennen.

Die Menge an eingesetztem Trockenmittel liegt im allgemeinen zwischen 0,1 und 20 Gew.-%, bevorzugt zwischen 0,5 und 15 Gew.-%, besonders bevorzugt zwischen 1,0 und 10 Gew.-%, bezogen auf die Gesamtmenge der öligen Suspension.

Das oder die verwendeten Trockenmittel können dabei auch separat - wie im Verfahrensschritt a) - in einem eßbaren Öl gemahlen werden und anschließend der öligen Suspension der gemahlenen wasserlöslichen Enzyme zugesetzt werden. Ferner ist es möglich, die Trockenmittel auch ungemahlen mit der öligen Suspension der gemahlenen wasserlöslichen Enzyme aus Verfahrensschritt a) zu mischen. Im Falle der Trockenmahlung können die wasserlöslichen Enzyme und das oder die Trockenmittel auch separat gemahlen und dann zur öligen Suspension gegeben werden.

Es wird überraschenderweise gefunden, daß die Zugabe von Trockenmitteln eine Verminderung der Viskosität der öligen Suspension im Vergleich zu trockenmittelfreien öligen Suspension bewirkt.

Aufgrund der feinteiligen Verteilung der dispergierten wasserlöslichen Enzyme zeichnen sich die nach dem erfindungsgemäßen Verfahren hergestellten öligen Suspensionen durch eine hohe Bioverfügbarkeit der in der Suspension enthaltenen Wirkstoffe aus.

Neben den eingangs genannten wasserlöslichen Enzymen können vor, während oder nach der Mahlung zusätzliche fettlösliche Vitamine, wie z.B. die K-Vitamine, Vitamin A und Derivate wie Vitamin A-Acetat, Vitamin A-Propionat oder Vitamin A-Palmitat, Vitamin D₂ und Vitamin D₃ sowie die bereits genannten E-Vitamine in die ölige Suspension eingetragen und gelöst werden. Bevorzugt erfolgt die Mahlung im Schritt a) sowie die Suspendierung im Schritt b) in Gegenwart fettlöslicher Vitamine.

Gegenstand der Erfindung sind auch ölige Suspensionen mindestens eines wasserlöslichen Enzyms, erhältlich nach dem erfindungsgemäßen, vorstehend beschriebenen Verfahren.

Die erfindungsgemäßen öligen Suspensionen enthalten dabei 1 bis 70 Gew.-%, bevorzugt 2 bis 60 Gew.-%, besonders bevorzugt 10 bis 55 Gew.-% ganz besonders bevorzugt 15 bis 50 Gew.-% mindestens eines der eingangs genannten wasserlöslichen Enzyme in feinstvermahlener Form.

Außerdem können die öligen Suspensionen zusätzlich 0,5 bis 60 Gew.-%, bevorzugt 5 bis 50 Gew.-%, besonders bevorzugt 10 bis 45 Gew.-%, ganz besonders bevorzugt 15 bis 40 Gew.-% mindestens eines der eingangs genannten fettlöslichen Vitamine in gelöster Form enthalten.

Darüberhinaus können die öligen Zubereitungen zusätzlich mindestens ein weiteres Carotinoid enthalten.

Unter Carotinoide sind z.B. folgende Verbindungen zu verstehen: β-Carotin, Lycopin, Lutein, Astaxanthin, Zeaxanthin, Cryptoxanthin, Citranaxanthin, Canthaxanthin, Bixin, β-Apo-4-carotinal, β-Apo-8-carotinal, β-Apo-8-carotinsäureester, einzeln oder als Mischung. Bevorzugt verwendete Carotinoide sind β-Carotin, Lycopin, Lutein, Astaxanthin, Zeaxanthin, Citranaxanthin und Canthaxanthin.

Die Carotinoide können dabei in kristalliner Form oder als Formulierung - beispielsweise als Trockenpulver, gemäß EP-A-0 065 193 eingesetzt werden.

Vorteilhafterweise werden die Carotinoide in der Regel in kristalliner Form zusammen mit den wasserlöslichen Enzymen in dem Öl gemahlen. Im Falle von Astaxanthin und Canthaxanthin werden bevorzugt Astaxanthin- bzw. Canthaxanthin-haltige Trockenpulver, beispielsweise Lucantin® Pink bzw. Lucantin® Rot (ein 10%iges Astaxanthin- bzw. Canthaxanthin-Trockenpulver, Fa. BASF Aktiengesellschaft, Ludwigshafen, Deutschland) zusammen mit den wasserlöslichen Enzymen eingesetzt.

Der Gehalt an Carotinoide liegt in den Formulierungen im allgemeinen zwischen 0,1 und 40 Gew.-%, bevorzugt zwischen 0,3 und 20 Gew.-%, besonders bevorzugt zwischen 0,5 und 10 Gew.-%, ganz besonders bevorzugt zwischen 1 und 5 Gew.-%, bezogen auf die Gesamtmenge der Formulierung.

Je nach Anwendungszweck können die erfindungsgemäßen öligen Zubereitungen bis zu 10 Gew.-% weiterer Zusatzkomponenten wie beispielsweise Mineralstoffe, Aminosäuren, Proteine oder fettlösliche Enzyme.

Diese Zusatzstoffe, ebenso wie die o.g. fettlöslichen Vitamine und Carotinoide, können vor, während oder nach der Mahlung der erfindungsgemäßen Suspension zugesetzt werden. Um eine möglichst feinteilige homogene Suspension aller nicht-öllöslicher Bestandteile zu erhalten, ist es von Vorteil, die o.g. Zusatzstoffe ebenfalls zusammen mit den wasserlöslichen Enzymen zu mahlen.

Als Mineralstoffe können beispielsweise Eisensulfat, Zinksulfat, Mangansulfat, Kupfersulfat, Calciumsulfat, Natriumsulfat, Kupferoxid, Magnesiumoxid, Calciumfluorid, Kaliumchlorid, Kaliumjodid, Natriumchlorid, Calciumjodat, Calcium-, Magnesium-, Kalium-, Natrium- oder Eisen-Phosphat, Cobaltcarbonat, Natriumselenat oder Kieselsäure und deren Salze in die Suspension eingearbeitet und mitvermahlen werden. Die Menge an eingesetzten Mineralstoffen, beispielsweise im Tierernährungsbereich, orientiert sich dabei jeweils am Bedarf der zu fütternden Tiere.

Als Aminosäurereste kommen generell alle bekannten physiologisch unbedenklichen α-Aminosäurereste in Frage. Bevorzugt zu nennen sind die Reste folgender Aminosäuren: Alanin, Arginin, Asparagin, Asparaginsäure, Cystein, Cystin, Glutamin, Glutaminsäure, Glycin, Histidin, Isoleucin, Leucin, Lysin, Methionin, Phenylalanin, Hippursäure, Serin und Taurin. Besonders bevorzugt sind Lysin, Methionin und Cystein.

### Weitere Bestandteile der Suspension können sein:

Verbindungen mit Vitamin- oder Coenzymcharakter, z.B. Cholinchlorid, Carnitin, γ-Butyrobetain, Liponsäure, Kreatin, Ubichinone, S-Methylmethionin, S-Adenosylmethionin.

Mehrfach ungesättigte Fettsäuren, z.B. Linolsäure, Linolensäure, Arachidonsäure, Eicosapentaensäure, Docosahexaensäure.

Fütterungsantibiotika für Medizinalfutter sowie Mikroorganismen zur Verbesserung der Verdauung.

In manchen Fällen kann es erforderlich sein, daß die öligen Suspensionen außerdem Hilfsstoffe, wie z.B. Schutzkolloide, Antioxidantien, Verdicker, Chelatbildner, wie z.B. Alkali- oder Erdalkalisalze der Citronensäure, Phytinsäure oder Phosphorsäure enthalten und/oder Emulgatoren enthalten.

Als Schutzkolloide können beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen.

Zur Erhöhung der Stabilität des Wirkstoffes gegen oxidativen Abbau ist es vorteilhaft, Stabilisatoren wie α-Tocopherol, t-Butyl-hydroxy-toluol, t-Butylhydroxyanisol, oder Ethoxyquin zuzusetzen.

Als Emulgatoren bzw. Solubilisatoren können beispielsweise Polyglycerin-Fettsäureester, Sorbitan-Fettsäureester, Propylenglycol-Fettsäureester oder Lecithin verwendet werden.

Die Suspensionen eignen sich u.a. als Zusatzstoff in Tierfuttermittelzubereitungen bzw. in Mischfutter und zur Herstellung von Nahrungsergänzungspräparaten im Tierbereich.

Bevorzugt lassen sich die Suspensionen als Futtermittelzusatz in der Tierernährung einsetzen, bevorzugt zum Auftragen bzw. Aufsprühen auf Futtermittelpellets, besonders bevorzugt zum Zumischen zu Futtermittelmischungen vor dem Pelletieren.

Die Anwendung als Futtermittelzusatzstoff erfolgt insbesondere durch direktes Aufsprühen der erfindungsgemäßen Suspensionen, gegebenenfalls nach Verdünnung mit Ölen, beispielsweise auf Tierfutterpellets als sogenannte "post-pelleting-application".

Eine bevorzugte Ausführungsform des Sprühverfahrens besteht darin, daß man die Futtermittelpellets unter vermindertem Druck mit der öligen Suspension belädt.

Beispiele hierfür finden sich u.a. in GB-A-2 232 573 sowie in EP-A-0 556 883.

Bevorzugt richtet sich die Erfindung auf Tierfuttermittel, insbesondere auf Futtermittelpellets, die mit den Suspensionen beladen werden oder vor dem Pelletieren der Futtermittelmischungen in gewünschten Kombinationen zugemischt werden.

Die nach dem erfindungsgemäßen Verfahren herstellbaren öligen Suspensionen weisen den Vorteil einer erhöhten Lagerstabilität auf und eine verminderte Anfälligkeit gegenüber mikrobiologischen Verkeimungen.

Zusätzlich können die öligen Suspensionen ohne den aufwendigen Verfahrensschritt der Umhüllung direkt den Futtermittelmischungen vor dem Pelletieren zugesetzt werden.

In den folgenden Beispielen wird die Herstellung der erfindungsgemäßen öligen Suspensionen wasserlöslicher Enzyme näher erläutert.

### Beispiel 1

Zwei Kilogramm einer Mischung aus 25 Gew.-% getrocknete Phytase (aus Aspergillus, sprühgetrocknet), und 75 Gew.-% Maiskeimöl werden mit einem Blattrührer solange gerührt, bis eine homogene Suspension vorliegt. Danach wird die Mischung in eine rührbare Vorlage umgefüllt, aus der die Suspension mittels Schlauchpumpe durch eine kontinuierlich betriebene Kugelmühle (Dyno Mill KDL Spezial) gefördert wird. Der Mahlbehälter der Kugelmühle ist mit 400 g Glaskugeln (Durchmesser 800 bis 1200 µm) gefüllt. Die aus der Mühle austretende feinteilige Suspension wird aufgefangen und mittels eines Partikelgrößenmeßgerätes (Malvern Mastersizer) vermessen. Der Mahlvorgang wird so oft wiederholt bis die suspendierten Teilchen eine mittlere Teilchengröße kleiner 20 µm haben.

## Patentansprüche

1. Verfahren zur Herstellung öliger Suspensionen wasserlöslicher Enzyme, **dadurch gekennzeichnet, dass** man in Abwesenheit wasserlöslicher Vitamine und in Gegenwart von Trockenmitteln
a) mindestens ein wasserlösliches Enzym in einem Öl bis auf eine mittlere Partikelgrösse von 0,1 bis 100 µm mahlt oder
b) mindestens ein wasserlösliches Enzym ohne Verwendung einer kontinuierlichen Phase bis auf eine mittlere Partikelgrösse von 0,1 bis 100 µm mahlt und die gemahlenen Partikel anschliessend in einem Öl suspendiert.

2. Verfahren nach Anspruch 1, **dadurch gekennzeichnet, dass** es sich bei dem Öl um mindestens ein essbares Öl handelt.

3. Verfahren nach einem der Ansprüche 1 oder 2, **dadurch gekennzeichnet, dass** es sich bei dem Öl um ein bei 20°C flüssiges Öl handelt.

4. Verfahren nach einem der Ansprüche 2 oder 3, **dadurch gekennzeichnet, dass** man als essbares Öl Vitamin E, Vitamin E Derivate oder Mischungen davon verwendet.

5. Verfahren nach einem der Ansprüche 1 bis 4, **dadurch gekennzeichnet, dass** die Mahlung im Schritt a) sowie die Mahlung und/oder Suspendierung in Schritt b) in Abwesenheit eines Emulgators erfolgen.

6. Verfahren nach einem der Ansprüche 1 bis 5, **dadurch gekennzeichnet, dass** die Mahlung im Schritt a) sowie die Mahlung und/oder Suspendierung in Schritt b) in Abwesenheit eines Schutzkolloids erfolgen.

7. Verfahren nach einem der Ansprüche 1 bis 6, **dadurch gekennzeichnet, dass** man mindestens ein wasserlösliches Enzym als Feststoff mit einem Wassergehalt kleiner 10 Gew.-% verwendet.

8. Verfahren nach einem der Ansprüche 1 bis 7, **dadurch gekennzeichnet, dass** die Mahlung im Schritt a) sowie die Suspendierung in Schritt b) in Gegenwart fettlöslicher Vitamine erfolgen.

9. Verfahren nach Anspruch 1, **dadurch gekennzeichnet, dass** man als Trockenmitteln Stoffe, ausgewählt aus der Gruppe, bestehend aus Alkali- und Erdalkalisulfaten, Alkali- und Erdalkalichloriden und Kieselgel verwendet.

10. Ölige Suspensionen mindestens eines wasserlöslichen Enzyms, erhältlich nach einem Verfahren, definiert gemäss einem der Ansprüche 1 bis 9.

11. Ölige Suspensionen nach Anspruch 10, enthaltend 1 bis 70 Gew.-% mindestens eines wasserlöslichen Enzyms.

12. Ölige Suspensionen nach einem der Ansprüche 10 oder 11, enthaltend zusätzlich 0,5 bis 60 Gew.-% mindestens eines fettlöslichen Vitamins.

13. Verwendung der öligen Suspensionen, definiert gemäss einem der Ansprüche 10 bis 12 als Zusatz zu Tierfuttermitteln.

14. Verwendung nach Anspruch 13 als Futtermittelzusatz in der Tierernährung.

15. Verwendung nach Anspruch 14 zum Auftragen auf Futtermittelpellets.

16. Verwendung nach Anspruch 14 zum Zumischen zu Futtermittelmischungen vor dem Pelletieren.

17. Verwendung nach Anspruch 15, **dadurch gekennzeichnet, dass** man die Futtermittelpellets unter vermindertem Druck mit der öligen Suspension belädt.

18. Verwendung einer öligen Suspension mindestens eines wasserlöslichen Enzyms mit einer mittleren Partikelgrösse von 0,1 bis 100 µm zum Auftragen auf Futtermittelpellets.

19. Futtermittelpellets, enthaltend ölige Suspensionen definiert gemäss einem der Ansprüche 10 bis 12
